(19) Europäisches Patentamt
European Patent Office
Office européen des brevets

(11) Publication number: 0 104 721 B1

(12) # EUROPEAN PATENT SPECIFICATION

(45) Date of publication of patent specification: **30.12.86**

(51) Int. Cl.$^4$: **C 07 D 307/14,** C 07 C 143/78

(21) Application number: **83304162.7**

(22) Date of filing: **18.07.83**

(54) **Process for the preparation of 2-chlorosulfonyl-4-(N-substituted sulfamyl)-chlorobenzene compounds.**

(30) Priority: **27.08.82 JP 147821/82**

(43) Date of publication of application: **04.04.84 Bulletin 84/14**

(45) Publication of the grant of the patent: **30.12.86 Bulletin 86/52**

(84) Designated Contracting States: **DE FR GB**

(56) References cited:
**GB-A- 909 661**
**GB-A-1 031 916**

(73) Proprietor: **FUJI YAKUHIN KOGYO KABUSHIKI KAISHA**
**530, Chokeiji**
**Takaoka-shi Toyama-ken (JP)**

(72) Inventor: **Nakahashi, Kazuaki**
**100-37, Ejira**
**Takaoka-shi Toyama-ken (JP)**
Inventor: **Miyamura, Souji**
**3-27, Yokota 2-chome**
**Takaoka-shi Toyama-ken (JP)**
Inventor: **Takeuchi, Tadashi**
**1-30, Shimotako**
**Himi-shi Toyoama-ken (JP)**

(74) Representative: **Allard, Susan Joyce et al**
**BOULT, WADE & TENNANT 27 Furnival Street**
**London EC4A 1PQ (GB)**

EP 0 104 721 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a new process for the preparation of 2-chlorosulfonyl-4-(N-substituted sulfamyl)-chlorobenzene compounds. More particularly, the present invention relates to a new industrially valuable process for the preparation of 2-chlorosulfonyl-4-(N-substituted sulfamyl)-chlorobenzene compounds in one stage from 2,4-dichlorosulfonyl-chlorobenzene.

2-Chlorosulfonyl-4-(N-substituted sulfamyl)-chlorobenzene compounds are known to be important as intermediates in the preparation of certain compounds having pharmacological activity. For example, 2-chlorosulfonyl-4-[N-methyl-N-(α-methyltetrahydrofurfuryl)-sulfamyl]-chlorobenzene of the formula:

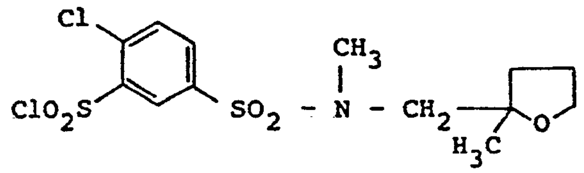

is useful as an intermediate in the preparation of a substance exhibiting excellent diuretic activity, i.e. this compound is extremely valuable as a precursor for 2-sulfamyl-4-[N-methyl-N-(α-methyl-tetrahydrofurfuryl)sulfamyl]-chlorobenzene of the formula:

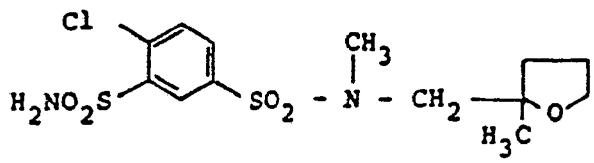

However, these 2-chlorosulfonyl-4-(N-substituted sulfamyl)-chlorobenzene compounds, the end products of the present invention, have heretofore been prepared by an industrially very disadvantageous method. Previously, it was practically impossible to effect amidation at the 4-position alone of 2,4-dichloro-sulfonyl-chlorobenzene. The amidation by ammonia or an amine of the halosulfonyl group of monochlorosulfonyl-chlorobenzene compounds is disclosed in GB 909661 and GB 1031916. These British patents do not, however, disclose the selective monoamidation of dichlorosulfonyl-chlorobenzenes.

In the prior art methods, therefore, 2-chlorosulfonyl-4-(N-substituted sulfamyl)-chlorobenzene compounds were prepared by incorporating at the 2-position of 4-chlorosulfonyl-chlorobenzene, a functional group which is not sensitive to amidation of the chlorosulfonyl group in the 4-position (i.e. sulfamylation), and then converting the functional group in the 2-position, after the amidation in the 4-position with an amine, to a chlorosulfonyl group by a multi-stage reaction including the Meerwein reaction.

These methods require several reaction vessels and are complicated and troublesome operations which are quite disadvantageous, particularly in that the overall yield of the end product does not reach a satisfactory level and the methods *per se* are not commercially attractive. There is thus a great demand in this art for developing a new advantageous method for preparing 2-chlorosulfonyl-4-(N-substituted sulfamyl)-chlorobenzene compounds in a high yield without the above-mentioned drawbacks.

We have now surprisingly found that 2-chlorosulfonyl-4-(N-substituted sulfamyl)-chlorobenzene can be successfully be prepared in a high yield in one step from 2,4-dichlorosulfonyl-chlorobenzene by selectively effecting amidation of the chlorosulfonyl group in the 4-position only.

In accordance with the present invention, there is provided a process for the preparation of 2-chlorosulfonyl-4-(N-substituted sulfamyl)-chlorobenzene compounds of the general formula:

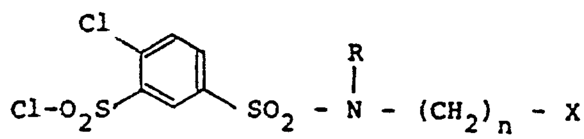

(I)

wherein R is a hydrogen atom or a lower (i.e. $C_1$—$C_6$) alkyl group X is a 5- or 6-membered alicyclic group which may contain one or two oxygen atoms as ring-constituting members and may be substituted on the ring by one or more lower (i.e. $C_1$—$C_6$) alkyl groups and *n* is 0 or 1, which process comprises reacting a 2,4-dichlorosulfonylchlorobenzene of the formula:

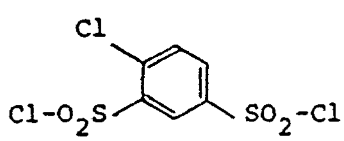

(II)

with an amine of the general formula:

$$R—NH—(CH_2)_n—X \quad (III)$$

wherein R, X and *n* are as defined above in the presence of an acid-binding agent at a temperature of below 10°C, preferably below 0°C and more preferably below −40°C.

Of the reactants used in the process of this invention, 2,4-dichlorosulfonyl-chlorobenzene of the formula (II) is commercially available and can easily be prepared by the chlorosulfonation of chlorobenzene using a conventional procedure. Primary or secondary amines of the general formula (III) are known and also commercially available. These amines can of course be prepared as required by methods known *per se*.

The group R is a hydrogen atom or a lower alkyl group which may be straight or branched chain and contains 1 to 6 carbon atoms. Examples of R as an alkyl group include methyl, ethyl, n-propyl, isopropyl, n-butyl, isobutyl, sec-butyl, tert-butyl, n-pentyl, isopentyl, n-hexyl and 2-ethylbutyl. The group X in principle contains no unsaturated double bond and is a $C_{5-6}$ alicyclic group which may contain one or two oxygen atoms as ring-constituting members in place of one or two ring carbon atoms and may be substituted by one or more, generally one or two, lower alkyl groups on the ring carbon atoms. This lower alkyl group as a ring substituent is of the same type as referred to above with respect to the group R. Examples of the group X thus include cyclopentyl, cyclohexyl, tetrahydrofurfuryl, tetrahydropyranyl and 1,4-dioxan-2-yl, as well as their ring-substituted lower alkyl homologs.

Any base usually employed for condensation reactions accompanied by the liberation of an acid can be used as the acid-binding agent in the process of this invention. Examples of suitable acid-binding agents are inorganic bases such as alkali metal hydroxides, for example, sodium hydroxide and potassium hydroxide, alkaline earth metal hydroxides, for example, calcium hydroxide and barium hydroxide, and alkali metal carbonates and bicarbonates, for example, sodium carbonate, potassium carbonate, sodium bicarbonate and potassium bicarbonate; and organic bases such as tertiary amines, for example, triethylamine, tri-n-propylamine, tri-n-butylamine, pyridine, picolines, lutidines and collidines, and quaternary ammonium hydroxides, for example, tetramethylammonium hydroxide. The use of a tertiary amine, especially triethylamine, is preferred because it forms a homogeneous system with the solvent used and the amine of the general formula (III).

Any organic solvent which is inert to the reaction can be used in the process of this invention. Illustrative of utilizable organic solvents are, for example, ethers such as diethyl ether, tetrahydrofuran and dioxane; ketones such as acetone, methyl ethyl ketone and methyl isobutyl ketone; esters such as methyl acetate, ethyl acetate and ethyl propionate; and organic tertiary bases such as pyridine, triethylamine and tri-n-propylamine. In general, polar solvents as exemplified above are preferred to non-polar solvents such as aromatic hydrocarbons, because the latter tend to involve disadvantages in after treatments. As organic tertiary bases such as pyridine or triethylamine function both as the acid-binding agent and solvent, a large excess amount of the organic tertiary base can be used for such dual purpose.

The reactants in the process of this invention, i.e., 2,4-dichlorosulfonyl-chlorobenzene and an amine of the general formula (III) are generally used in a stoichiometric amount. However, either of the reactants may be used in a slight excess amount. The acid-binding agent is generally used in a stoichiometric amount necessary to combine with the acid liberated during the condensation reaction of the reactants. When an organic tertiary base is used it may be used in a large excess amount so that it is also effective as the organic solvent.

The process of the present invention is generally carried out by adding a solution of an amine of the general formula (III) and the acid-binding agent in an organic solvent to a solution of 2,4-dichlorosulfonyl-chlorobenzene in the organic solvent and the mixture is then stirred until the condensation reaction is completed. The use of the same organic solvent to dissolve the reactants is convenient to simplify the aftertreatment.

The above condensation reaction should be carried out at a temperature below 10°C, preferably 0°C and more preferably −40°C. The establishment of this low reaction temperature is the most important feature of the present invention. Under these low temperature conditions, the 4-chlorosulfonyl group alone of the starting 2,4-dichlorosulfonyl-chlorobenzene is selectively amidated but the 2-chlorosulfonyl group is not attacked, or even if it is attacked, the resultant 2-amidated and/or 2,4-diamidated product is present in an extremely small aamount and can easily be separated and eliminated from the desired product after the reaction. The formation of such by-products becomes negligible as the reaction temperature adopted is lowered. Accordingly, the reaction can advantageously be conducted at a temperature below 0°C, preferably −40°C. As the amidation takes place selectivey in the 4-position of the starting 2,4-dichlorosulfonyl-chlorobenzene, the product of general formula (I) is obtained not only in a very high yield but also very pure.

After completion of the reaction, the reaction mixture is treated in a conventional manner to isolate the product of general formula (I) in a pure state. For example, the reaction mixture may be concentrated to dryness under reduced pressure whereby the solvent used is recovered. The residue is then purified by column chromatography or preparative thin layer chromatography using, for example, silica gel as sorption agent followed by recrystallization from benzene-n-hexane. If necessary, the residue may be

subjected to a preliminary purifying treatment prior to the chromatographic purification, which comprises taking up the residue in a water-immiscible organic solvent, washing the solution with water several times and then removing the solvent under reduced pressure. Such a preliminary purifying treatment is sometimes necessary when an inorganic acid-binding agent is used in the main reaction step. The thus purified product of general formula (I) is usually in the form of a white crystalline powder.

It is indeed a most important advantage of the present invention that the amidation reaction takes place selectively in the 4-position only of the starting 2,4-dichlorosulfonyl-chlorobenzene so that the product of general formula (I) can be obtained in a high yield and with good purity. Furthermore, some additional advantages can also be achieved by the present invention. First, the product produced by the present invention represented by the general formula (I) can be further reacted, without the necessity of isolation from the reaction liquid, directly with ammonia or a substance capable of producing ammonia, such as ammonium hydroxide or ammonium carbonate, whereby the 2-chlorosulfonyl group in the compound of the general formula (I) can readily be converted into a 2-sulfamyl group to produce a compound of the general formula:

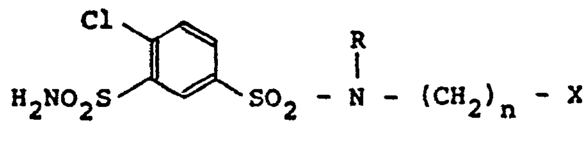

(IV)

wherein R, X and *n* are as defined above, which is useful as a diuretic. Thus, the process of this invention is of important utility from an industrial point of view. Secondly, the process of this invention is advantageous in that a pharmacologically useful sulfamyl compound of the general formula (IV), for example, 2-sulfamyl-4-[N-methyl-M-(α-methyltetrahydrofurfuryl)-sulfamyl]-chlorobenzene can be prepared economically in 3 steps starting from chlorobenzene and in only 2 steps starting from 2,4-dichlorosulfonylchlorobenzene. In addition, a pharmacologically valuable compound of the general formula (IV) can be obtained, as described above, from the product of the present invention, i.e. 2-chlorosulfonyl-4-[N-substituted sulfamyl)chlorobenzene of the general formula (I) by the treatment described above.

A great economic advantage can thus be achieved by the process of the present invention.

The present invention will now be illustrated in more detail by way of examples and reference examples. In the reference examples, a series of reactions starting from 2,4-dichlorosulfonyl-chlorobenzene for preparing pharmacologically valuable compounds are illustrated wherein the process of the present invention is included as the former half of the reaction. In laboratory scale reactions illustrated in these examples and reference examples, a temperature as low as −69°C of the solution was made by external cooling of the reaction vessels containing the solution with a mixture of trichloroethylene and dry ice as the coolant. A mixture of dry ice and ethanol may also be used as the coolant.

## Example 1

Preparation of 2-chlorosulfonyl-4-[N-methyl-N-(α-methyltetrahydrofurfuryl)-sulfamyl]-chlorobenzene

2-Methyltetrahydrofurfuryl-methylamine (1.3 g) and triethylamine (1.2 g) were dissolved in ethyl acetate (5 ml). This solution was added to a solution which had been prepared by dissolving 2,4-dichlorosulfonyl-chlorobenzene (4.4 g) in ethyl acetate (25 ml) and cooled at −68°C. After completion of the reaction, the solvent was distilled off under reduced pressure and the residue was subjected to column chromatography for purification and then recrystallized from benzene-n-hexane whereby the title compound was obtained as a white crystalline powder in a yield of 3.9 g (97% of the theoretical amount). M.P. 75.8°C Elementary analysis as $C_{13}H_{17}Cl_2NO_5S_2$ (M.W. 402.31)

Calc: S, 15.94%; Found: S, 15.92%

## Example 2

Preparation of 2-chlorosulfonyl-4-[N-(α-methyl-tetrahydrofurfuryl)-sulfamyl]-chlorobenzene

N-Methyltetrahydrofurfurylamine (1.2 g) and triethylamine (1.2 g) were dissolved in ethyl acetate (5 ml). This solution was added to a solution which had been prepared by dissolving 2,4-dichlorosulfonyl-chlorobenzene (4.4 g) in ethyl acetate (25 ml) and cooled at −69°C. After completion of the reaction, the solvent was distilled off under reduced pressure and the residue was subjected to column chromatography for purification and then recrystallized from benzene-n-hexane whereby 2-chlorosulfonyl-4-[N-(α-methyltetrahydrofurfuryl)-sulfamyl]-chlorobenzene was obtained as a white crystalline powder in a yield of 3.3 g (85%).

## Example 3

Preparation of 2-chlorosulfonyl-4-(N-methyl-N-cyclohexyl)-sulfamyl-chlorobenzene

N-Methylcyclohexylamine (3.4 g) and triethylamine (3.4 g) were dissolved in ethyl acetate (12 ml). This solution was added to a solution which had been prepared by dissolving 2,4-dichlorosulfonyl-chlorobenzene (14.0 g) in ethyl acetate (93 ml) and then cooled at −69°C. After completion of the reaction, the solvent was distilled off under reduced pressure and the residue was subjected to column

chromatography for purification and then recrystallized from benzene-n-hexane whereby 2-chlorosulfonyl-4-(N-methyl-N-cyclohexyl)-sulfamyl-chlorobenzene was obtained as a white crystalline powder in a yield of 11.0 g (95%).

Reference Example 1

Preparation of 2-sulfamyl-4-[N-methyl-N-(α-methyltetrahydrofurfuryl)-sulfamyl]-chlorobenzene

2-Methyltetrahydrofurfuryl-methylamine (3.9 g) and triethylamine (3.4 g) were dissolved in ethyl acetate (18 ml). This solution was added to a solution which had been prepared by dissolving 2,4-dichlorosulfonylchlorobenzene (14.0 g) in ethyl acetate (70 ml) and then cooled at −40°C to effect reaction. After completion of the reaction, 28% ammonia water (28.0 ml) was added at −40°C to the reaction mixture and then the temperature of the mixture was elevated up to −5°C. The reaction mixture was then allowed to stand for phase separation and the aqueous layer was discarded. The organic layer was concentrated under reduced pressure to distill off the solvent and the residue was crystallized from methanol whereby the title compound was obtained as a white crystalline powder in a yield of 9.3 g (80%). M.P. 149°C.

Reference Example 2

Preparation of 2-sulfamyl-4-[N-methyl-N-(α-methyltetrahydrofurfuryl)-sulfamyl]-chlorobenzene

2-Methyltetrahydrofurfuryl-methylamine (3.9 g) and triethylamine (3.4 g) were dissolved in acetone (15 ml). This solution was added to a solution which had been prepared by dissolving 2,4-dichlorosulfonyl-chlorobenzene (12.6 g) in acetone (100 ml) and cooled at −69°C to effect reaction. After completion of the reaction, 28% ammonia water (25.0 ml) was added at −69°C to the reaction mixture and the temperature of the mixture was then elevated up to 0°C. The reaction liquid was diluted with water and then extracted with ethyl acetate. The organic phase was concentrated under reduced pressure to distill off the solvent. The residue was purified to obtain the title compound. Yield 9.1 g (80%); M.P. 148°C.

Reference Example 3

Preparation of 2-sulfamyl-4-[N-methyl-N-(α-methyltetrahydrofurfuryl)-sulfamyl]-chlorobenzene

2,4-Dichlorosulfonyl-chlorobenzene (9.3 g) was dissolved in ethyl acetate (65 ml) and the solution was cooled to −69°C. Besides this, 2-methyltetrahydrofurfuryl-methylamine (2.6 g) and acid binding agent were dissolved in ethyl acetate (15 ml), and the solution was added slowly to the aforesaid ethyl acetate solution cooled to −69°C. After one hour, 28% ammonia water (16 ml) was slowly added dropwise to the reaction mixture at −69°C. The temperature of the mixture was elevated up to 5°C in about one hour. After completion of the reaction, the mixture was allowed to stand for phase separation and the aqueous layer was discarded. The organic layer was concentrated under reduced pressure until dryness. The caramel-like residue was dissolved in 3N-NaOH solution (35 ml) and the insoluble matters were removed by extraction with toluene (40 ml). The aqueous phase was neutralized with 6N-HCl solution (15 ml) and the white oily substance precipitated out was extracted with ethyl acetate (70 ml). The organic phase was decolorized, dried and concentrated until dryness. The residue thus obtained was then recrystallized from aqueous methanol (15 ml) whereby the title compound was obtained as a white crystalline powder in a yield of 6.9 g (90%). M.P. 149°C.

Reference Example 4

Preparation of 2-sulfamyl-4-[N-methyl-N-(α-methyltetrahydrofurfuryl)-sulfamyl]-chlorobenzene

2-Methyltetrahydrofurfuryl-methylamine (4.7 g) and triethylamine (4.9 g) were dissolved in tetrahydrofuran (16 ml). This solution was added to a solution which had been prepared by dissolving 2,4-dichlorosulfonyl-chlorobenzene (17.0 g) in tetrahydrofuran (170 ml) and cooled at −69°C to effect reaction. 28% Ammonia water (16.5 ml) was then added to the reaction liquid at −69°C. The temperature of the reaction mixture was allowed to rise up to −10°C and the mixture was extracted with ethyl acetate. The solvents were removed by distillation under reduced pressure and the residue was crystallized from methanol whereby the title compound was obtained as a white crystalline powder in a yield of 6.9 g (94%). M.P. 149°C.

Reference Example 5

Preparation of 2-sulfamyl-4-[N-(α-methyltetrahydrofurfuryl)-sulfamyl]-chlorobenzene

N-Methyltetrahydrofurfurylamine (1.2 g) and triethylamine (1.2 g) were dissolved in ethyl acetate (5 ml), and the solution was added to a solution which had been prepared by dissolving 2,4-dichlorosulfonyl-chlorobenzene (4.4 g) in ethyl acetate (25 ml) and cooled at −69°C to effect reaction. 28% Ammonia water (8.5 ml) was added to the reaction liquid kept at the same temperature and the temperature of the mixture was then elevated up to 10°C. The aqueous phase was removed by phase separation and the organic phase was concentrated under reduced pressure to remove the solvent. The residue was purified by column chromatography whereby 2-sulfamyl-4-[N-(α-methyl-tetrahydrofurfuryl)-sulfamyl]-chlorobenzene was obtained as a white crystalline powder in a yield of 2.8 g (80%). M.P. 162.4°C. Elementary analysis as $C_{12}H_{17}ClN_2O_5S_2$ (M.W. 368.85).

Calc.: S, 17.38%; Found: S, 17.12%

**Claims**

1. A process for the preparation of 2-chlorosulfonyl-4-(N-substituted sulfamyl)-chlorobenzene compounds of the general formula:

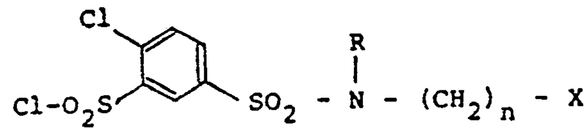

(I)

wherein R is a hydrogen atom or a $C_1$—$C_6$ alkyl group, X is a 5- or 6-membered alicyclic group which may contain one or two oxygen atoms as ring-constituting members and may be substituted on the ring by one or more $C_1$—$C_6$ alkyl groups, and *n* is 0 or 1, which process comprises reacting a 2,4-dichlorosulfonylchlorobenzene of the formula:

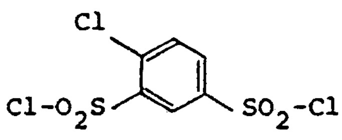

(II)

with an amine of the general formula:

$$R—NH—(CH_2)_n—X \qquad (III)$$

wherein, R, X and *n* are as defined above, in the presence of an acid-binding agent at a temperature of below 10°C.

2. A process as claimed in claim 1 wherein the temperature is maintained below 0°C.

3. A process as claimed in claim 2 wherein the temperature is maintained below −40°C.

4. A process as claimed in any one of the preceding claims wherein the reaction between the 2,4-dichlorosulfonylchlorobenzene and the amine is carried out in the presence of an organic solvent inert to the reaction.

5. A process as claimed in claim 4 wherein the solvent is an ether, ester, ketone or tertiary organic base.

6. A process as claimed in any one of the preceding claims wherein the acid-binding agent is an alkali metal hydroxide, an alkaline earth metal hydroxide, an alkali metal carbonate or bicarbonate, or a tertiary amine.

7. A process for the preparation of 2-sulfamyl-4-(N-substituted sulfamyl)-chlorobenzenes of the formula

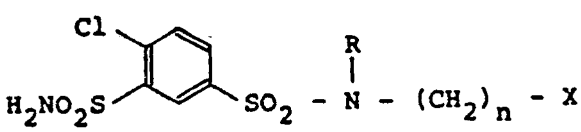

wherein R, X and n are as defined in claim 1, which process comprises:

(a) carrying out a process as defined in any one of claims 1 to 6, and

(b) reacting the resultant 2-chlorosulfonyl-4-(N-substituted sulfamyl)-chlorobenzene compound with ammonia or a substance capable of producing ammonia.

8. A process as claimed in claim 7 wherein step (b) is carried out without isolating the 2-chlorosulfonyl-4-(N-substituted sulfamyl)-chlorobenzene compound from the reaction liquid of step (a).

9. A process as claimed in claim 7 wherein the 2-chlorosulfonyl-4-(N-substituted sulfamyl)-chlorobenzene compound produced in step (a) is isolated prior to step (b) being carried out.

10. A process as claimed in any one of claims 7 to 9 wherein the substance capable of producing ammonia is ammonium hydroxide or ammonium carbonate.

**Patentansprüche**

1. Verfahren zur Herstellung von 2-Chlorsulfonyl-4-(N-substituierten Sulfamyl)-chlorbenzolverbindungen der allgemeinen Formel

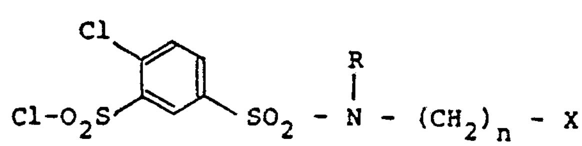

(I)

worin R ein Wasserstoffatom oder eine $C_1$—$C_6$-Alkylgruppe, X eine 5- oder 6-gliedrige alicyclische Gruppe,

die ein oder zwei Sauerstoffatome im Ring enthalten kann und die am Ring durch eine oder mehrere $C_1$—$C_6$-Alkylgruppen substituiert sein kann, und *n* 0 oder 1 bedeutet, wobei ein 2,4-Dichlorsulfonylchlorbenzol der Formel

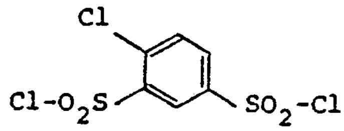

(II)

mit einem Amin der allgemeinen Formel

$$R—NH—(CH_2)_n—X \qquad (III),$$

worin R, X und *n* wie vorstehend definiert sind, in Gegenwart eines säurebindenden Mittels bei einer Temperatur von unterhalb 10°C umgesetzt werden.

2. Verfahren nach Anspruch 1, worin die Temperature unterhalb 0°C gehalten wird.

3. Verfahren nach Anspruch 2, worin die Temperatur unterhalb −40°C gehalten wird.

4. Verfahren nach einem der vorhergehenden Ansprüche, worin die Reaktion zwischen dem 2,4-Dichlorsulfonylchlorbenzol und dem Amin in Gegenwart eines gegenüber der Reaktion inerten organischen Lösungsmittel durchgeführt wird.

5. Verfahren nach Anspruch 4, worin das Lösungsmittel einen Äther, Ester, ein Keton oder eine tertiäre organische Base darstellt.

6. Verfahren nach einem der vorhergehenden Ansprüche, worin das säurebindende Mittel ein Alkalihydroxid, ein Erdalkalihydroxid, ein Alkalicarbonat oder -bicarbonat oder ein tertiäres Amin darstellt.

7. Verfahren zur Herstellung von 2-Sulfamyl-4-(N-substituierten Sulfamyl)-chlorbenzolen der Formel

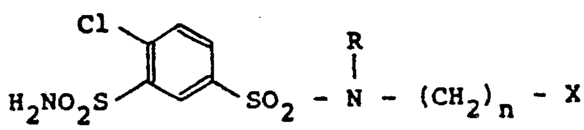

worin R, X und n wie in Anspruch 1 definiert sind, worin

(a) ein Verfahren, wie in einem der Ansprüche 1 bis 6 definiert, durchgeführt wird und

(b) die erhaltene 2-Chlorsulfonyl-4-(N-substituierte Sulfamyl)-chlorbenzolverbindung mit Ammoniak oder einer Substanz, die in der Lage ist, Ammoniak zu erzeugen, umgesetzt wird.

8. Verfahren nach Anspruch, 7, worin die Stufe (b) ohne Isolierung der 2-Chlorsulfonyl-4-(N-substituierten Sulfamyl)-chlorbenzolverbindung aus der Reaktionsflüssigkeit von Stufe (a) durchgeführt wird.

9. Verfahren nach Anspruch 7, worin die in Stufe (a) hergestellte 2-Chlorsulfonyl-4-(N-substituierte Sulfamyl)-chlorbenzolverbindung vor der Stufe (b) isoliert wird.

10. Verfahren nach einem der Ansprüche 7 bis 9, worin die Substanz, die in der Lage ist, Ammoniak zu erzeugen, Ammoniumhydroxid oder Ammoniumcarbonate darstellt.

**Revendications**

1. Procédé de préparation de composés 2-chlorosulfonyl-4-(sulfamyl-N-substitué)-chlorobenzènes de formule générale:

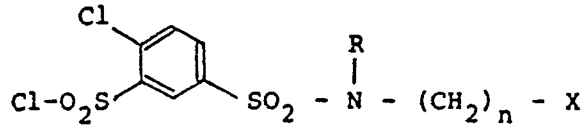

(I)

dans laquelle R est un atome d'hydrogène ou un groupe alkyle en $C_{1-6}$, X est un groupe alicyclique à 5 ou 6 chaînons pouvant contenir un ou deux atomes d'oxygène à titre de chaînons constitutifs du cycle et pouvant être substitué sur le cycle par un ou plusieurs groupes alkyle en $C_{1-6}$, et n vaut 0 ou 1, procédé qui comporte la réaction d'un 2,4-dichlorosulfonyl-chlorobenzène de formule:

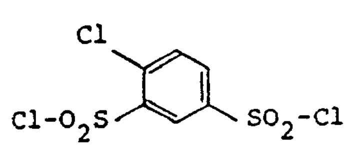

(II)

avec une amine de formule générale:

$$R{-}NH{-}(CH_2)_n{-}X$$

dans laquelle R, X et *n* sont tels que définis plus haut, en présence d'un agent de fixation d'acide, à une température inférieure à 10°C.

2. Procédé selon la revendication 1, dans lequel on maintient la température au-dessous de 0°C.

3. Procédé selon la revendication 2, dans lequel on maintient la température au-dessous de −40°C.

4. Procédé selon l'une quelconque des revendications précédentes, dans lequel on effectue la réaction entre le 2,4-dichloro-sulfonyl-chlorobenzène et l'amine en présence d'un solvant organique inerte vis-à-vis de la réaction.

5. Procédé selon la revendication 4, dans lequel le solvant est un éther, un ester, une cétone ou une base organique tertiaire.

6. Procédé selon l'une quelconque des revendications précédentes, dans lequel l'agent de fixation d'acide est un hydroxyde de métal alcalin, un hydroxyde de métal alcalino-terreux, un carbonate ou bicarbonate de métal alcalin, ou une amine tertiaire.

7. Procédé de préparation de 2-sulfamyl-4-(sulfamyl-N-substitué)chlorobenzène de formule:

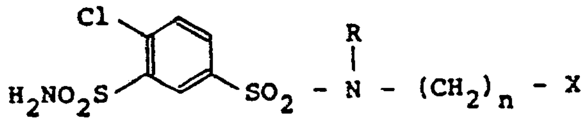

dans laquelle R, X et *n* sont tels que définis dans la revendication 1, procédé qui comporte;

(a) la réalisation du procédé défini dans l'une quelconque des revendications 1 à 6, et

(b) la réaction du composé 2-chlorosulfonyl-4-(sulfamyl-N-substitué)chlorobenzène avec de l'ammoniac ou une substance capable de produire de l'ammoniac.

8. Procédé selon la revendication 7, dans lequel on effectue l'étape (b) sans isoler le composé 2-chlorosulfonyl-4-(sulfamyl-N-substitué)chlorobenzène du liquide réactionnel de l'étape (a).

9. Procédé selon la revendication 7, dans lequel on isole le composé 2-chlorosulfonyl-4-(sulfamyl-N-substitué)chlorobenzène produit dans l'étape (a) avant d'effectuer l'étape (b).

10. Procédé selon l'une quelconque des revendications 7 à 9, dans lequel la substance capable de produire de l'ammoniac est l'hydroxyde d'ammonium ou le carbonate d'ammonium.